Europäisches Patentamt

European Patent Office

Office européen des brevets

(19)

(11) Publication number: **0 221 699**
**B1**

# (12) EUROPEAN PATENT SPECIFICATION

(45) Date of publication of the patent specification:
**10.01.90**

(51) Int. Cl.⁴: **A61K 31/71, A61K 31/34**

(21) Application number: **86307918.2**

(22) Date of filing: **14.10.86**

(54) Composition for the treatment of infections.

(30) Priority: **19.10.85 GB 8525829**
**19.10.85 GB 8525830**

(43) Date of publication of application:
**13.05.87 Bulletin 87/20**

(45) Publication of the grant of the patent:
**10.01.90 Bulletin 90/2**

(84) Designated Contracting States:
**BE CH DE ES FR GB GR IT LI LU NL SE**

(56) References cited:
**DE-A- 2 421 273**
**GB-A- 901 273**

**Garrod, Lambert, O'Grady: Antibiotic and
Chemotherapy, 3rd. Edition, Edinburgh (1971),
p. 270-273
V. Lorian et al. (eds.): Antibiotics in Laboratory
Medicine, (1980) p. 309-311
E. Jawetz: The Use of Combinations of Antimicrobial
Drugs. Annual Review of Pharmacology, Vol. 8 (1968),
p. 151-170
A. Manten, M.J. Wissen: Antagonism between
antibacterial drugs. Nature, Nr. 4803 (1961) p. 671-672**

(73) Proprietor: **BEECHAM GROUP PLC, Beecham House
Great West Road, Brentford Middlesex TW8 9BD(GB)**

(72) Inventor: **Wylie, David Ferguson Beecham
Pharmaceuticals, Walton Oaks Dorking Road, Tadworth
Surrey, KT20 7NT(GB)**
Inventor: **Poulton, Barbara Ann Beecham
Pharmaceuticals, Walton Oaks Dorking Road, Tadworth
Surrey, KT20 7NT(GB)**

(74) Representative: **Lockwood, Barbara Ann et al, Beecham
Pharmaceuticals Biosciences Research Centre Great
Burgh Yew Tree Bottom Road, Epsom Surrey
KT18 5XQ(GB)**

ACTORUM AG

## Description

The present invention relates to a novel composition for the treatment of bacterial and/or mycoplasmal infections.

Tylosin is known as an antibacterial/antimycoplasmal agent for use in the treatment of bacterial and mycoplasmal infections in animals.

The applicants have found a composition having improved antibacterial and antimycoplasmal properties.

According to the present invention there is provided a pharmaceutical composition comprising tylosin or a pharmaceutically acceptable salt thereof and a β-lactam antibiotic.

Preferably the β-lactum antibiotic is amoxycillin.

Advantageously the pharmaceutically acceptable salt of tylosin is tylosin tartrate.

The composition is preferably presented so as to facilitate concurrent administration of the tylosin or salt thereof and the β-lactam antibiotic.

For example, the tartrate salt of tylosin can be presented with a β-lactam antibiotic in a form suitable for administration in aqueous solution, for example in drinking water for animals.

When the β-lactam antibiotic is amoxycillin, suitable formulations include those known for administration by injection such as those described in GB-A-1,578,337 and GB-A-1,532,993.

The tartrate salt of tylosin, unlike tylosin itself, is water-soluble. However the dissolution characteristics of the tartrate salt are rather poor.

The applicants have found that by combining tylosin tartrate with an acidifying agent, a composition is produced which has substantially improved dissolution rates as compared to tylosin tartrate alone.

The tylosin tartrate/acidifying agent composition is suitably in the form of a concentrated aqueous dispersion, eg solution, or a dry powder for solution in drinking water. When the composition is in the form of a dry powder a concentrated dispersion is suitably prepared from it before addition to the drinking water.

The concentrated dispersion is suitably from 200 ml to 2 litres in volume.

Suitably the composition is supplied in the form of a dry powder in a container, the volume of which is greater than the concentrated dispersion to be prepared. The concentrated dispersion can then be prepared by filling the container with water to the required level and shaking for, for example two minutes. Once complete dispersion, eg dissolution, has occurred, the contents of the container can be poured into a conventional animal drinking water tank.

The amount of acidifying agent present should preferably be sufficient to establish a pH of from 2-6 in the concentrated dispersion.

Suitable acidifying agents include acid salts such as potassium dihydrogen phosphate and acids such as citric acid. The acid salts of alkali metals are preferred. The anion may be inorganic such a phosphate or organic such as citrate. The acidifying agent may be anhydrous or it may be a hydrate.

One suitable acidifying agent is disodium hydrogen citrate. In this instance the weight ratio of tylosin tartrate to disodium hydrogen citrate in the composition is suitably from 1:1 to 100:1 such as about 23:1.

Another suitable acidifying agent is citric acid. In this instance the weight ratio of tylosin tartrate to citric acid in the composition is suitably from 1:1 to 125:1 such as about 30:1 or 7:3.

The quantity of tylosin tartrate present is such that the concentration of tylosin in the final solution is that conventionally employed in therapy. For example for use in animal drinking water, a concentration of tylosin of approximately 100-1000 ppm (or 8-80 mg/kg body weight), more preferably approximately 200-1000 ppm, most preferably about 500 ppm can be employed.

The above described tylosin tartrate composition forms a complete clear solution of tylosin at a rapid rate. No precipitation is observed even when hard water is employed in the formation of the solution.

The ratio of tylosin tartrate to β-lactam antibiotic in the composition of the invention will depend upon the particular β-lactam antibiotic employed. Suitably the β-lactam antibiotic is present in the solution in the conventional dosages. When the antibiotic is amoxycillin the ratio of tylosin tartrate: β-lactam antibiotic is suitably from 10:1 to 0.1:1 more suitably from 10:1 to 0.5:1.

Many β-lactam antibiotics including amoxycillin require high pH to facilitate solubility in water.

The applicants have found that by combining amoxycillin with a basic salt such as trisodium phosphate, a composition is produced which has advantageous dissolution characteristics.

Suitable basic salts include sodium glycine carbonate and trisodium phosphate. The basic salts of alkali metals are preferred. The anion may by inorganic such as phosphate or carbonate or it may be organic.

The amoxycillin/basic salt composition is suitably in the form of a concentrated aqueous solution or a dry powder for solution in drinking water. When the composition is in the form of a dry powder a concentrated solution is suitably prepared from it before addition to the drinking water. Typically the concentrated solution, which is suitably from 60 ml to 1 litre in volume, will be added to the drinking water concurrently with the concentrated dispersion of tylosin tartrate.

Suitably the composition is supplied in the form of a dry powder in a container, the volume of which is greater than the concentrated solution to be prepared. The concentrated solution can then be prepared by filling the container with water to the required level and shaking for, for example, 2 minutes. Once dissolution has occurred, the contents of the container can be poured into a conventional animal drinking water tank.

The amount of basic salt present should preferably be sufficient to establish a pH of at least about 9, preferably 9.5, in the concentrated solution. Suitably the composition consists of one part by weight of trisodium phosphate to about one part by weight of amoxycillin.

A small amount of a sequestering agent may also be included in the amoxycillin composition, such as ethylenediamine tetraacetate or a salt thereof such as its disodium salt (disodium edetate).

A particularly preferred composition comprises about 11 parts amoxycillin trihydrate, 10 parts trisodium phosphate, and 1 part disodium edetate.

The concentration of amoxycillin in the final solution, such as animal drinking water, is suitably approximately 50-500 ppm (or 4-40 mg/kg bodyweight), most preferably about 250 ppm.

It has been found that since soluble tylosin compositions require acid pH conditions, and many β-lactam antibiotics including amoxycillin require high pH to facilitate solubility, it is not possible to mix two such compositions together directly.

Further according to the present invention there is provided a container having two compartments, one of said compartments containing tylosin tartrate and an acidifying agent and the second of said compartments containing a β-lactam antibiotic adapted for dissolution in water, such as the above-mentioned amoxycillin/trisodium phosphate composition.

Still further according to the present invention there is provided animal drinking water containing tylosin tartrate and a β-lactam antibiotic.

Advantageously, where the β-lactam antibiotic is amoxycillin, the pH of the animal drinking water is in the range of from 5.2 to 6.8, preferably about 6.3, in order to optimize the stability of the amoxycillin. In order to achieve this, the above-mentioned acidifying agent is selected so as to serve both to solubilize the tylosin tartrate and to ensure the correct pH in the final solution.

The invention will now be particularly described by way of example with reference to the accompanying drawings in which:

Figure 1 is a schematic side view of a container of the invention; and

Figure 2 is a schematic side view of an alternative embodiment of a container of the invention.

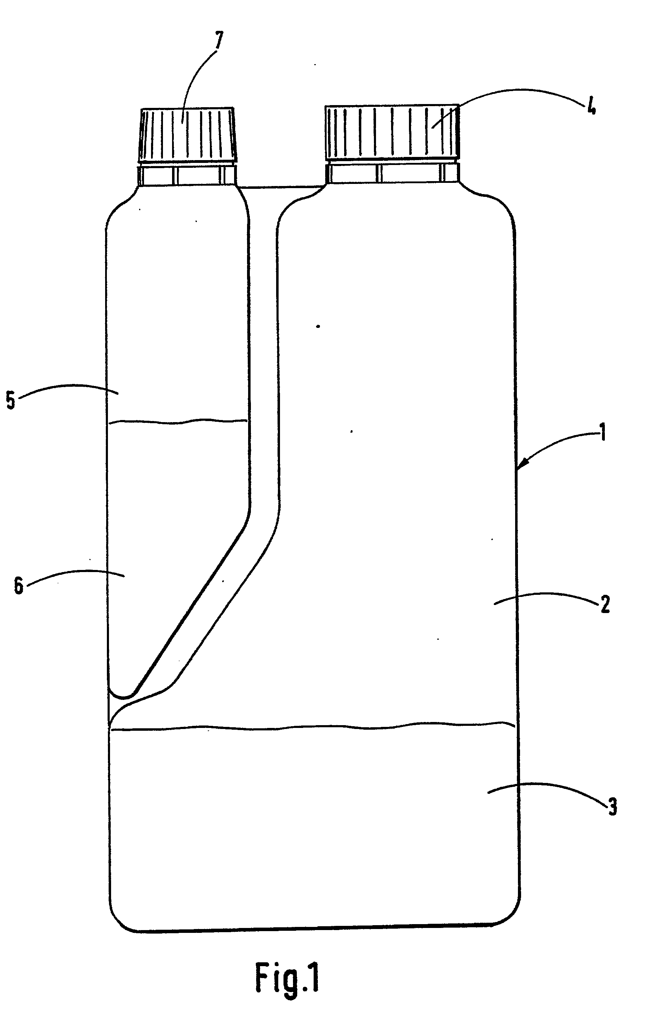

The container 1 shown in figure 1 comprises a first compartment 2 having a capacity of at least 700mls. Inside the compartment 2 is a mixture 3 comprising 120g of tylosin tartrate and 5.26g of disodium hydrogen citrate in dry powder form. The compartment 2 is provided with a screw cap 4.

Also within the container 1 is a compartment 5 having a volume of at least 120ml. Within the compartment 5 is 32g of an amoxycillin formulation 6 in dry powder form. The amoxycillin formulation 6 comprises 51.00% by weight amoxycillin trihydrate, 4.54% by weight disodium edetate and 44.46% by weight trisodium phosphate. Compartment 5 is provided with a separate screw cap 7.

The container 1 is suitably made of a plastics material such as high density polyethylene or polypropylene. It is intended that the container 1 should be supplied to a farmer. In use, the compartments 2, 5 are first partly filled with water and the contents briefly shaken until they are completely dispersed.

The contents of both compartments 2 and 5 are then emptied into the drinking water tank of the animals, which will typically contain about 200 litres of drinking water. The pH of the dilute solution obtained is then 7.8.

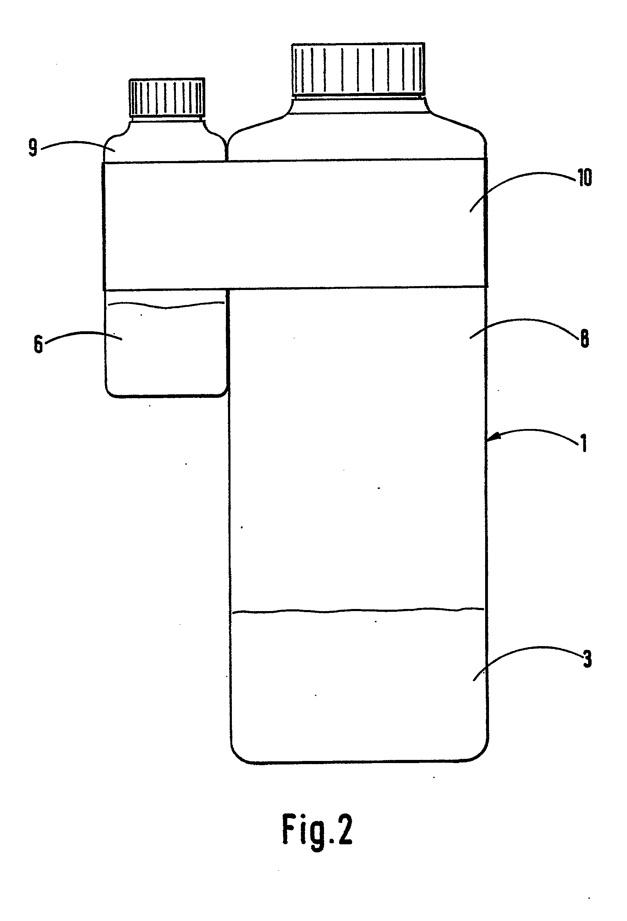

In the alternative embodiment shown in figure 2, the compartments 2,5 are replaced by a first and a second bottle 8 and 9 respectively. The bottles 8,9 are suitably of plastics material. They are held together for example by means of a strap 10 as shown or alternatively by fusion or welding. The volume of the bottles 8,9 can be varied depending upon the amount of composition contained therein. For example, the first bottle 8 can be approximately 1 litre in volume whereas the second bottle 9 is 125ml in capacity.

In a further alternative embodiment of the invention, the compartment 5 or the bottle 9 has a capacity of at least 350ml and contains 98.1g of the amoxycillin formulation 6, whilst the compartment 2 or the bottle 8 has a capacity of at least 700ml and contains 120g tylosin tartrate and 50g citric acid, which replaces the disodium hydrogen citrate of the first embodiment. The contents of the container 1 are mixed with water as set out above and then emptied into about 200 litres of animal drinking water. The pH of the dilute solution obtained is 6.3.

## Claims for the contracting states: BE, CH, DE, FR, GB, IT, LI, LU, NL, SE

1. A pharmaceutical product containing tylosin or a pharmaceutically acceptable salt thereof and a β-lactam antibiotic, for concurrent use in the treatment of bacterial and/or mycoplasmal infections.

2. A product according to claim 1, comprising tylosin tartrate and a β-lactam antibiotic in water-soluble form.

3. An aqueous solution of the product according to claim 2.

4. A product according to claim 3, wherein the β-lactam antibiotic is amoxycillin and the aqueous solution water has a pH in the range 5.2 to 6.8.

5. A product according to claim 2, comprising a first container containing tylosin tartrate and an acidifying agent and a second container containing a β-lactam antibiotic in water-soluble form, the contents of each container being in dry powder form or aqueous dispersion.

6. A product according to claim 5, wherein the contents of one or each container are in dry powder form and the capacity of the container is sufficient to contain an aqueous dispersion of the whole of the contents.

7. A product according to claim 5 or 6, wherein the acidifying agent is disodium hydrogen citrate or citric acid.

8. A product according to claim 5, 6, or 7, wherein the second container contains amoxycillin and trisodium phosphate.

9. A product according to any one of claims 5 to 8, wherein the β-lactam antibiotic is amoxycillin and the weight ratio of tylosin tartrate to amoxycillin is in the range 10:1 to 0.1:1.

10. Use of tylosin or a pharmaceutically acceptable salt thereof and a β-lactam antibiotic for the manufacture of a product according to any preceding claim.

**Claims for the contracting states: ES, GR**

1. Use of tylosin or a pharmaceutically acceptable salt thereof and a β-lactam antibiotic for the manufacture of a pharmaceutical product for use in the treatment of bacterial and/or mycoplasmal infections.

2. Use according to claim 1, wherein the product comprises tylosin tartrate and a β-lactam antibiotic in water-soluble form.

3. Use according to claim 2 wherein the product is an aqueous solution.

4. Use according to claim 3, wherein the β-lactam antibiotic is amoxycillin and the aqueous solution has a pH in the range 5.2 to 6.8.

5. Use according to claim 2, wherein the product comprises a first container containing tylosin tartrate and an acidifying agent and a second container containing a β-lactam antibiotic in water-soluble form, the contents of each container being in dry powder form or aqueous dispersion.

6. Use according to claim 5, wherein the contents of one or each container are in dry powder form and the capacity of the container is sufficient to contain an aqueous dispersion of the whole of the contents.

7. Use according to claim 5 or 6, wherein the acidifying agent is disodium hydrogen citrate or citric acid.

8. Use according to claim 5, 6, or 7, wherein the second container contains amoxycillin and trisodium phosphate.

9. Use according to any one of claims 5 to 8, wherein the β-lactam antibiotic is amoxycillin and the weight ratio or tylosin tartrate to amoxycillin is in the range 10:1 to 0.1:1.

**Patentansprüche für die Vertragsstaaten: BE, CH, DE, FR, GB, IT, LI, LU, NL, SE**

1. Ein pharmazeutisches Produkt, enthaltend Tylosin oder ein pharmazeutisch zulässiges Salz davon und ein ß-Lactam-Antibiotikum, für die zusammenwirkende Behandlung von bakteriellen und/oder durch Mykoplasmen hervorgerufenen Infektionen.

2. Ein Produkt gemäß Anspruch 1, umfassend Tylosin-Tartrat und ein ß-Lactam-Antibiotikum in wasserlöslicher Form.

3. Eine wäßrige Lösung des Produktes gemäß Anspruch 2.

4. Ein Produkt gemäß Anspruch 3, in welchem das ß-Lactam-Antiobiotikum Amoxycillin ist und die wäßrige Lösung einen pH-Wert im Bereich von 5,2 bis 6,8 hat.

5. Ein Produkt gemäß Anspruch 2, umfassend einen ersten Behälter, der Tylosin-Tartrat und ein ansäuerndes Mittel enthält, sowie einen zweiten Behälter, welcher ein ß-Lactam-Antibiotikum in wasserlöslicher Form enthält, wobei der Inhalt jedes Behälters in Form eines trockenen Pulvers oder einer wäßrigen Dispersion vorliegt.

6. Ein Produkt nach Anspruch 5, bei welchem der Inhalt eines oder jedes Behälters in Form eines trockenen Pulvers vorliegt und das räumliche Fassungsvermögen der Behälter ausreicht, um eine wäßrige Dispersion der gesamten Inhalte aufzunehmen.

7. Ein Produkt nach Anspruch 5 oder 6, bei welchem das ansäuernde Mittel Di-Natrium-hydrogencitrat oder Zitronensäure ist.

8. Ein Produkt nach Anspruch 5, 6 oder 7, bei welchem der zweite Behälter Amoxycillin und Trinatriumphosphat enthält.

9. Ein Produkt nach irgendeinem der Ansprüche 5 bis 8, bei welchem das ß-Lactam-Antibiotikum Amoxycillin ist und das Gewichtsverhältnis von Tylosin-Tartrat zu Amoxycillin im Bereich von 10:1 bis 0,1:1 liegt.

10. Verwendung von Tylosin oder eines pharmazeutisch zulässigen Salzes davon und eines ß-Lactam-Antibiotikums zur Herstellung eines Produktes gemäß irgendeinem der vorhergehenden Ansprüche.

**Patentansprüche für die Vertragsstaaten: ES, GR**

1. Verwendung eines pharmazeutischen Produktes, enthaltend Tylosin oder ein pharmazeutisch zulässiges Salz davon und ein β-Lactam-Antibiotikum, für die zusammenwirkende Behandlung von bakteriellen und/oder durch Mycoplasmen hervorgerufenen Infektionen.

2. Verwendung gemäß Anspruch 1, wobei das Produkt Tylosin-Tartrat und ein β-Lactam-Antibiotikum in wasserlöslicher Form enthält.

3. Verwendung gemäß Anspruch 2, wobei das Produkt eine wäßrige Lösung ist.

4. Verwendung gemäß Anspruch 3, wobei das β-Lactam-Antibiotikum Amoxycillin ist und die wäßrige Lösung einen pH-Wert im Bereich von 5,2 bis 6,8 aufweist.

5. Verwendung gemäß Anspruch 2, wobei das Produkt einen ersten Behälter umfaßt, der Tylosin-Tartrat und ein ansäuerndes Mittel enthält, sowie einen zweiten Behälter, welcher ein β-Lactam-Antibiotikum in wasserlöslicher Form enthält, wobei der Inhalt jedes Behälters in Form eines trockenen Pulvers oder einer wäßrigen Dispersion vorliegt.

6. Verwendung nach Anspruch 5, wobei der Inhalt eines oder jeden Behälters in Form eines trockenen Pulvers vorliegt und das räumliche Fassungsvermögen der Behälter ausreicht, um eine wäßrige Dispersion des gesamten Inhalts aufzunehmen.

7. Verwendung gemäß Anspruch 5 oder 6, wobei das ansäuernde Mittel Di-Natrium-hydrogencitrat oder Zitronensäure ist.

8. Verwendung gemäß Anspruch 5, 6 oder 7, wobei der zweite Behälter Amoxycillin und Trinatriumphosphate enthält.

9. Verwendung nach irgendeinem der Ansprüche 5 bis 8, wobei daß β-Lactam-Antibiotikum Amoxycillin ist und das Gewichtsverhältnis von Tylosin-Tartrat zu Amoxycillin im Bereich von 10:1 bis 0,1:1 liegt.

**Revendications pour les états contractants: BE, CH, DE, FR, GB, IT, LI, LU, NL, SE**

1. Produit pharmaceutique, caractérisé en ce qu'il contient de la tylosine ou un sel de celle-ci acceptable du point de vue pharmaceutique et un antibiotique à cycle β-lactame utiles conjointement dans le traitement d'infections bactériennes et/ou mycoplasmiques.

2. Produit suivant la revendication 1, caractérisé en ce qu'il comprend le tartrate de tylosine et un antibiotique à cycle β-lactame sous une forme soluble dans l'eau.

3. Solution aqueuse de produit suivant la revendication 2.

4. Produit suivant la revendication 3, caractérisé en ce que l'antibiotique à cycle β-lactame est l'amoxycilline et que la solution aqueuse à un pH dans la gamme de 5,2 à 6,8.

5. Produit suivant la revendication 2, caractérisé en ce qu'il comprend un premier récipient contenant du tartrate de tylosine et un agent acidifiant et un second récipient contenant un antibiotique à cycle β-lactame sous une forme soluble dans l'eau, le contenu de chaque récipient étant sous la forme de poudre sèche ou de dispersion aqueuse.

6. Produit suivant la revendication 5, caractérisé en ce que le contenu d'un ou de chaque récipient est sous forme de poudre sèche et que la capacité du récipient est suffisante pour contenir une dispersion aqueuse de la totalité des contenus.

7. Produit suivant la revendication 5 ou 6, caractérisé en ce que l'agent acidifiant est du citrate acide disodique ou de l'acide citrique.

8. Produit suivant les revendications 5, 6 ou 7, caractérisé en ce que le second récipient contient de l'amoxycilline et du phosphate tricalcique.

9. Produit suivant l'une quelconque des revendications 5 à 8, caractérisé en ce que l'antibiotique à cycle β-lactame est l'amoxycilline et que le rapport pondéral du tartrate de tylosine à l'amoxycilline est dans la gamme de 10:1 à 0,1:1.

10. Utilisation de la tylosine ou d'un sel de celle-ci acceptable du point de vue pharmaceutique et d'un antibiotique à cycle β-lactame pour la fabrication d'un produit suivant l'une quelconque des revendications précédentes.

**Revendications pour les états contractants: ES, GR**

1. Utilisation de la tylosine ou d'un sel de celle-ci acceptable du point de vue pharmaceutique et d'un antibiotique à cycle β-lactame pour la fabrication d'un produit pharmaceutique utile dans le traitement d'infections bactériennes et/ou mycoplasmiques.

2. Utilisation suivant la revendication 1, caractérisée en ce que le produit comprend du tartre de tylosine et un antibiotique à cycle β-lactame sous une forme soluble dans l'eau.

3. Utilisation suivant la revendication 2, caractérisée en ce que le produit est une solution aqueuse.

4. Utilisation suivant la revendication 3, caractérisée en ce que l'antibiotique à cycle β-lactame est l'amoxycilline et que la solution aqueuse a un pH dans la gamme de 5,2 à 6,8.

5. Utilisation suivant la revendication 2, caractérisée en ce que le produit comprend un premier récipient contenant du tartrate de tylosine et un agent acidifiant et un second récipient contenant un antibiotique à cycle β-lactame sous une forme soluble dans l'eau, le contenu de chaque récipient étant sous forme de poudre sèche ou de dispersion aqueuse.

6. Utilisation suivant la revendication 5, caractérisée en ce que le contenu de l'un ou de chacun des récipients est sous la forme d'une poudre sèche et que la capacité du récipient est suffisante pour contenir une dispersion aqueuse de la totalité des contenus.

7. Utilisation suivant la revendication 5 ou 6, caractérisée en ce que l'agent acidifiant est du citrate acide disodique ou de l'acide citrique.

8. Utilisation suivant les revendications 5, 6 ou 7, caractérisée en ce que le second récipient contient de l'amoxycilline et du phosphate trisodique.

9. Utilisation suivant l'une quelconque des revendications 5 à 8, caractérisée en ce que l'antibiotique à cycle β-lactame est l'amoxycilline et que le rapport pondéral du tartrate de tylosine à l'amoxycilline est dans la gamme de 10:1 à 0,1:1.

Fig.1

9

10

6

8

1

3

Fig.2